# EUROPÄISCHE PATENTSCHRIFT

(11) **EP 1 748 730 B1**
(45) Veröffentlichungstag und Bekanntmachung des Hinweises auf die Patenterteilung: **02.03.2011**
(21) Anmeldenummer: 05744915.9
(22) Anmeldetag: 04.05.2005
(51) Int. Cl.: A61B 6/08, A61B 6/00

(54) **RÖNTGENEINRICHTUNG, INSBESONDERE MAMMOGRAPHIE-RÖNTGENEINRICHTUNG, MIT INDIKATORMITTELN IN FORM VON LEDS**
X-RAY APPARATUS, EXPECIALLY MOMMOGRAPHIC X-RAY APPARATUS, COMPRISING INDICATING MEANS IN THE FORM OF LEDS
DISPOSITIF A RAYONS X, EN PARTICULIER DISPOSITIF DE MAMMOGRAPHIE AUX RAYONS X, COMPRENANT DES MOYENS D'INDICATIONS SOUS LA FORME DE LED

(30) Priorität: 11.05.2004 DE 102004023046
(43) Veröffentlichungstag der Anmeldung: 07.02.2007
(73) Patentinhaber: SIEMENS AKTIENGESELLSCHAFT, 80506 München (DE)
(72) Erfinder: BRANDSTÄTTER, Werner, 90427 Nürnberg (DE); RAMSAUER, Martin, 90602 Pyrbaum (DE)
(86) Internationale Anmeldenummer: PCT/EP2005/052047
(87) Internationale Veröffentlichungsnummer: WO 2005/110233

(56) Entgegenhaltungen:
- EP-A- 0 819 407
- DE-A1- 19 943 898
- US-A- 4 825 455
- US-A- 5 563 924
- US-A- 6 036 362
- US-A- 6 104 778
- US-B1- 6 305 842

## Beschreibung

Die Erfindung betrifft eine Röntgeneinrichtung, insbesondere eine Mammographie-Röntgeneinrichtung, gemäß Oberbegriff des Patentanspruches 1; eine derartige Röntgeneinrichtung ist aus der DE 199 43 898 A1 bekannt.

Mammographie-Röntgeneinrichtungen besitzen Indikatormittel, durch die vor dem eigentlichen Vorgang der Röntgenaufnahme das Röntgenstrahlfeld auf der Oberfläche eines Patienten und/oder auf einem Objekttisch überprüft wird, um zum Beispiel sicherzustellen, dass die richtige Blende gewählt wurde. Als Indikatormittel ist dabei üblicherweise eine Glühbirne vorgesehen, die seitlich des Röntgenstrahlengangs gehaltert ist. Der zunächst zum Röntgenstrahl senkrecht verlaufende Lichtstrahl der Glühbirne wird durch einen im Röntgenstrahlengang angeordneten Spiegel in die Strahlrichtung des Röntgenstrahles umgelenkt. Bei der eigentlichen Röntgenaufnahme wird der Spiegel aus dem Strahlengang geklappt oder er verbleibt dort, sofern er röntgenstrahltransparent ist.

Aus der DE 199 43 898 A1 sind, insbesondere für als Operationshilfe dienende Röntgeneinrichtungen, Indikatormittel in Form von Laserdioden bekannt, die entweder am Röntgendetektor oder an der Röntgenquelle angebracht sind, um den von Röntgenstrahlung durchsetzten Bereich oberhalb und/oder das Röntgenstrahlfeld auf der Oberfläche des Patienten sichtbar zu machen. Die Indikatormittel werden an die Geometrie des Röntgenstrahles, insbesondere die Größe des Öffnungswinkels, anpassbar ausgestaltet, beispielsweise dadurch dass ein Signal zur Veränderung der Blendenöffnung der Blende an die Indikatormittel weitergegeben wird.

Der vorliegenden Erfindung liegt die Aufgabe zugrunde, bei einer derartigen Röntgeneinrichtung, insbesondere einer Mammographie-Röntgeneinrichtung, auf aufwandsarme Weise die der Röntgenaufnahme vorausgehende Ausleuchtung des Röntgenstrahlfeldes zu vereinfachen.

Die Lösung dieser Aufgabe ist bei einer Röntgeneinrichtung, insbesondere einer Mammographie-Röntgeneinrichtung, gemäß O-berbergriff des Patentanspruches 1 durch die kennzeichnende Lehre möglich; vorteilhafte Ausgestaltungen sind jeweils Gegenstand der zugehörigen Unteransprüche.

Die erfindungsgemäße Röntgeneinrichtung, insbesondere Mammographie-Röntgeneinrichtung, bietet durch die Anbringung der Indikatormittel zwischen Röntgenquelle und Blende und durch die umlenkungsfreie Ausrichtung des Ausleuchtstrahls den Vorteil, dass die Blende nicht nur zur Strahlformung des Röntgenstrahles und zur Strahlformung des Ausleuchtstrahles mitbenutzt wird sondern auch auf eine Umlenkung des Ausleuchtstrahles verzichtet werden kann; eine aufwändige Steuerung der Ausrichtung der Indikatormittel entfällt und ein umklappbarer Spiegel erübrigt sich, was einen kompakten und wartungsarmen Aufbau ermöglicht.

In für eine kompakte und einfache Bauweise der Röntgeneinrichtung vorteilhafter Weise ist die zumindest eine LED an einer der Röntgeneinrichtung zugeordneten, zwischen Röntgenquelle und Blende befindlichen Filteranordnung gehaltert. Da die Filteranordnung in den meisten Röntgeneinrichtungen grundsätzlich vorhanden ist, ist lediglich eine Halterung der LED notwendig.

Die Erfindung sowie weitere vorteilhafte Ausgestaltungen gemäß Merkmalen der Unteransprüche werden im folgenden anhand schematisch dargestellter Ausführungsbeispiele in der Zeichnung näher erläutert; es zeigen:
FIG 1 in Seitenansicht eine bekannte Mammographie-Röntgeneinrichtung mit Glühbirne und Umlenkspiegel zur Ausleuchtung eines Röntgenstrahlfeldes;
FIG 2 in Seitenansicht eine erfindungsgemäße Mammographie-Röntgeneinrichtung mit mindestens einer LED auf einer Filteranordnung zur Ausleuchtung des Röntgenstrahlfeldes;
FIG 3 in Detailansicht eine Filteranordnung gemäß FIG 2 mit mindestens einer LED.

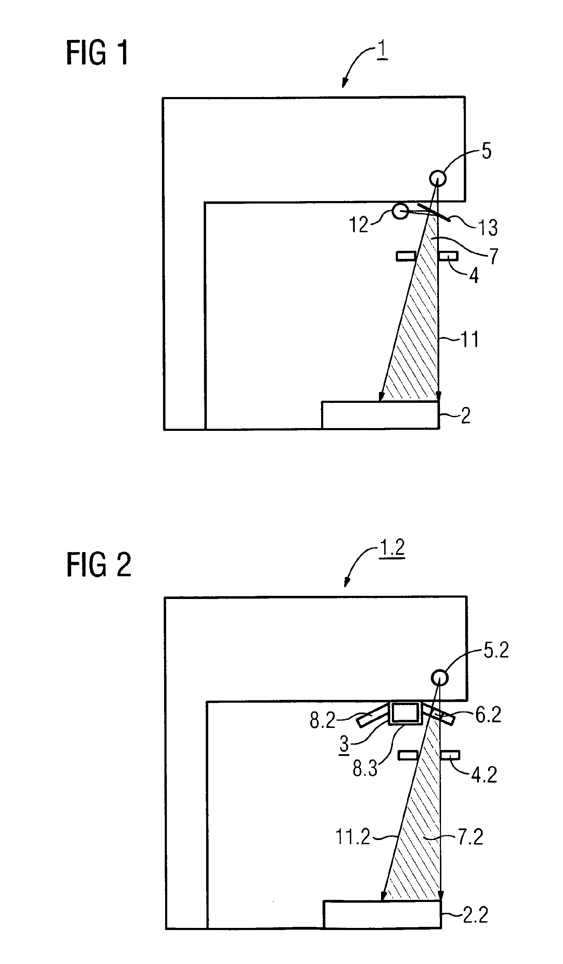

FIG 1 zeigt eine bekannte Mammographie-Röntgeneinrichtung 1, die als wesentliche Bestandteile eine Röntgenquelle 5 sowie einen Detektor, insbesondere einen mit einem Röntgenfilm bestückten Objekttisch 2, enthält. Im Untersuchungsfall wird durch die Röntgenquelle 5 ein Röntgenstrahl 11 erzeugt, der zur Abbildung eines nicht explizit gezeigten Untersuchungsobjektes eines Patienten auf den Detektor dient. Eine Blende 4 ist derart angeordnet, dass sie den Röntgenstrahl 11 durch Ausblendung von Teilbereichen eingrenzen kann. Vor dem eigentlichen Vorgang der Röntgenaufnahme wird ein Röntgenstrahlfeld auf der Oberfläche des Patienten oder auf dem Objekttisch 2 mittels eines von einer Glühbirne 12 erzeugten und durch einen Spiegel 13 in Richtung auf das Röntgenstrahlfeld umgelenkten Ausleuchtstrahles 7 überprüft. Die Glühbirne 12 und der Spiegel 13 sind derart eingestellt, dass der Ausleuchtstrahl 7 und der Röntgenstrahl 11 auf der Oberfläche des Patienten bzw. dem Objekttisch 2 im wesentlichen deckungsgleich sind. Der Spiegel 13 ist, um den Röntgenstrahl 11 nicht zu behindern, röntgenstrahltransparent oder aus dem Röntgenstrahl 11 klappbar.

FIG 2 zeigt eine erfindungsgemäße Röntgeneinrichtung 1.2 mit der Halterung mindestens einer LED 6.2 an einer an sich vorhandenen Filteranordnung 3. Für eine größere Helligkeit können auch mehrere LEDs vorgesehen sein. Die Filteranordnung 3, die zwischen der Röntgenquelle 5.2 und der Blende 4.2 angebracht ist, dient üblicherweise dazu, einzelne Filter 8.1; 8.2; 8.3 zur Ausfilterung von für die jeweilige Röntgenaufnahme nicht benötigten Frequenzen in den Röntgenstrahl zu bringen. Die zumindest eine LED 6.2 ist in vorteilhafter Weise in der Filteranordnung 3, insbesondere anstelle eines Filters 8.1; 8.2; 8.3, positionierbar und aus dem Röntgenstrahl 11.2 heraus verschwenkbar angeordnet. Zur Ausleuchtung des Röntgenstrahlfeldes wird die Filteranordnung 3 so verschwenkt, dass sich die mindestens eine LED 6.2 exakt im Strahlengang des Röntgenstrahles 11.2 befindet und der Ausleuchtstrahl 7.2 sich im wesentlichen mit dem Röntgenstrahl 11.2 deckt. Der Ausleuchtstrahl 7.2 ist dann umlenkungsfrei auf das Röntgenstrahlfeld gerichtet. Umlenkungsfrei schließt dabei nicht die Verwendung von Linsen zur Korrektur des Ausleuchtstrahles im Bereich bis 15° aus. Der Ausleuchtstrahl 7.2 wird durch die Positionierung der LED zwischen der Röntgenquelle 5.2 und der Blende 4.2 von dieser derart eingegrenzt, dass das Strahlfeld des Ausleuchtstrahles auf der Oberfläche des Patienten und/oder dem Objekttisch 2.2 mit dem Röntgenstrahlfeld des Röntgenstrahles 11.2 im wesentlichen deckungsgleich ist. Zweckmäßigerweise ist die mindestens eine LED 6.2 durch Drehen der Filteranordnung 3 um ihre Längsachse aus dem Röntgenstrahl 11.2 heraus verschwenkbar. Für die Röntgenaufnahme ist anstelle der LED der benötigte Filter (8.1; 8.2; 8.3) in den Strahlengang schwenkbar.

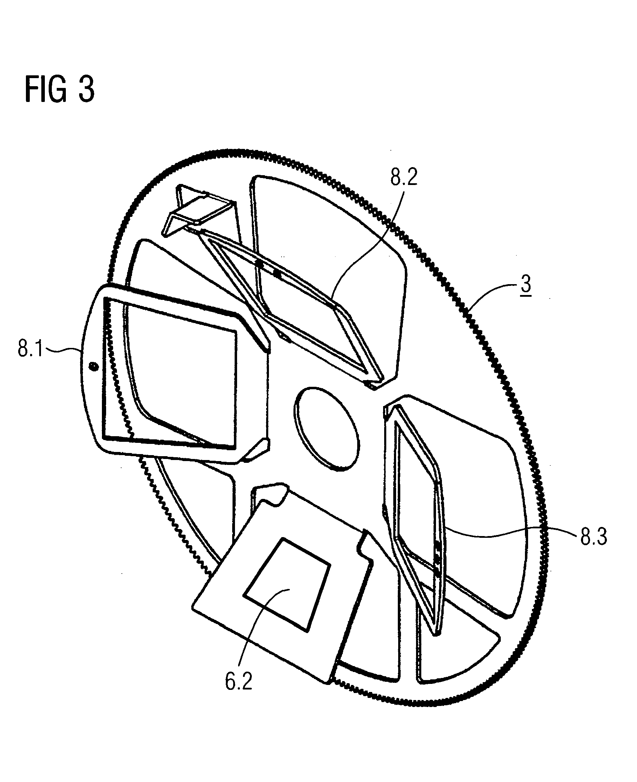

FIG 3 zeigt eine detaillierte Ansicht einer in FIG 2 angedeuteten Filteranordnung 3 mit vier für Filter vorgesehenen Halterungen, drei in Halterungen eingesetzten einzelnen Filtern (8.1; 8.2; 8.3) und in einer Halterung anstelle eines Filters angeordneter mindestens einer LED 6.2.

Die Erfindung lässt sich wie folgt kurz zusammenfassen: Zur aufwandsarmen Ausleuchtung eines Röntgenstrahlfeldes auf der Oberfläche eines Patienten ist bei einer Röntgeneinrichtung, insbesondere einer Mammographie-Röntgeneinrichtung, mit einem von einer Röntgenquelle erzeugten und von einer Blende eingrenzbaren Röntgenstrahl als Indikatormittel zumindest eine LED zwischen der Röntgenquelle und der Blende angeordnet, der Ausleuchtstrahl der zumindest einen LED umlenkungsfrei auf das Röntgenstrahlfeld gerichtet und die zumindest eine LED aus dem Röntgenstrahl heraus verschwenkbar; nach einer Ausführung der Erfindung ist die zumindest eine LED an einer zwischen Röntgenquelle und Blende angebrachten Filteranordnung gehaltert.

## Patentansprüche

1. Röntgeneinrichtung, insbesondere Mammographie-Röntgeneinrichtung, mit einem von einer Röntgenquelle (5.2) erzeugten und von einer Blende (4.2) eingrenzbaren Röntgenstrahl (11.2) zur Erstellung von Röntgenbildern eines Patienten und mit einem von Indikatormitteln in Form von zumindest einer LED (6.2) erzeugten Ausleuchtstrahl (7.2) zur Ausleuchtung eines entsprechenden Röntgenstrahlfeldes auf der Oberfläche des Patienten, **dadurch gekennzeichnet,**
- **dass** die zumindest eine LED (6.2) zwischen der Röntgenquelle (5.2) und der Blende (4.2) angeordnet ist und der Ausleuchtstrahl (7.2) der LED (6.2) umlenkungsfrei auf das Röntgenstrahlfeld gerichtet ist; **dadurch gekennzeichnet,**
- **dass** die zumindest eine LED (6.2) aus dem Röntgenstrahl (11.2) heraus verschwenkbar ist.

2. Röntgeneinrichtung nach Anspruch 1 mit einer Filteranordnung (3) zwischen der Röntgenquelle (5.2) und der Blende (4.2), **dadurch gekennzeichnet,**
**dass** die zumindest eine LED (6.2) an der Filteranordnung (3) gehaltert ist.

3. Röntgeneinrichtung nach Anspruch 2, **dadurch gekennzeichnet,**
**dass** die zumindest eine LED (6.2) in der Filteranordnung (3), insbesondere an Stelle eines Filters (8.1; 8.2; 8.3), positionierbar und aus dem Röntgenstrahl (11.2) heraus verschwenkbar angeordnet ist.

4. Röntgeneinrichtung nach Anspruch 3, **dadurch gekennzeichnet,**
**dass** die zumindest eine LED (6.2) durch Drehen der Filteranordnung (3) um ihre Längsachse aus dem Röntgenstrahl (11.2) heraus verschwenkbar ist.

## Claims

1. X-ray apparatus, more particularly a mammography X-ray apparatus, with an X-ray beam (11.2) for generating X-ray images of a patient, which X-ray beam is generated by an X-ray source (5.2) and can be delimited by a diaphragm (4.2), and with an illumination beam (7.2) for illuminating a corresponding X-ray beam field on the surface of the patient, which illumination beam is generated by indicator means in the form of at least one LED (6.2), **characterized**
- **in that** the at least one LED (6.2) is arranged between the X-ray source (5.2) and the diaphragm (4.2) and the illumination beam (7.2) from the LED (6.2) is directed without deflections at the X-ray beam field;
- **in that** the at least one LED (6.2) can be pivoted out of the X-ray beam (11.2).

2. X-ray apparatus according to Claim 1, with a filter arrangement (3) between the X-ray source (5.2) and the diaphragm (4.2), **characterized**
**in that** the at least one LED (6.2) is mounted on the filter arrangement (3).

3. X-ray apparatus according to Claim 2, **characterized in that** the at least one LED (6.2) is arranged such that it can be positioned in the filter arrangement (3), more particularly in place of a filter (8.1; 8.2; 8.3), and can be pivoted out of the X-ray beam (11.2).

4. X-ray apparatus according to Claim 3, **characterized in that** the at least one LED (6.2) can be pivoted out of the X-ray beam (11.2) by rotating the filter arrangement (3) about the longitudinal axis thereof.

## Revendications

1. Dispositif à rayons X, notamment dispositif à rayons X de mammographie, comprenant un rayonnement ( 11.2 ) X produit par une source ( 5.2 ) de rayons X et pouvant être délimité par un diaphragme ( 4.2 ), pour l'élaboration de radiographies d'un patient et comprenant un rayonnement ( 7.2 ) d'éclairage produit par des moyens indicateurs sous la forme d'au moins une DEL ( 6.2 ) pour éclairer un champ de rayonnement X correspondant sur la surface du patient, **caractérisé,**
- **en ce que** la au moins une DEL ( 6.2 ) est disposée entre la source ( 5.2 ) de rayons X et le diaphragme ( 4.2 ) et le rayonnement ( 7.2 ) d'éclairage de la DEL ( 6.2 ) est dirigé sans déviation sur le champ de rayonnement X,
- **en ce que** la au moins une DEL ( 6.2 ) peut basculer en-dehors du rayonnement ( 11.2 ) X.

2. Dispositif à rayons X suivant la revendication 1, comprenant un dispositif ( 3 ) de filtrage entre la source ( 5.2 ) de rayons X et le diaphragme ( 4.2 ), **caractérisé,**
**en ce que** la au moins une DEL ( 6.2 ) est fixée sur le dispositif ( 3 ) de filtrage.

3. Dispositif à rayons X suivant la revendication 2, **caractérisé,**
**en ce que** la au moins une DEL ( 6.2 ) peut être mise en position dans le dispositif ( 3 ) de filtrage, notamment à la place d'un filtre ( 8.1 ; 8.2 ; 8.3 ), et peut basculer hors du rayonnement ( 11.2 ) X.

4. Dispositif à rayons X suivant la revendication 3, **caractérisé,**
**en ce que** la au moins une DEL ( 6.2 ) peut être basculée hors du rayonnement ( 11.2 ) X en faisant tourner le dispositif ( 3 ) de filtrage autour de son axe longitudinal.
